# EUROPEAN PATENT APPLICATION

(11) **EP 4 643 913 A1**
(43) Date of publication of application: **05.11.2025**
(21) Application number: 23912553.7
(22) Date of filing: 09.11.2023
(51) Int. Cl.: A61M 15/00, A24F 40/20, A24F 40/05, A24F 40/42

(54) **INHALER**

(30) Priority: 30.12.2022 KR 20220190082
(71) Applicant: KT&G Corporation, Daedeok-gu Daejeon 34337 (KR)
(72) Inventor: KIM, Moon Won, Daejeon 34128 (KR); JUNG, Yong Mi, Daejeon 34128 (KR); SHIN, Jun Won, Daejeon 34128 (KR)
(74) Representative: Ter Meer Steinmeister & Partner
(86) International application number: PCT/KR2023/017954
(87) International publication number: WO 2024/143875

(57) **Abstract**

An inhaler for inhalation of a functional material, according to an embodiment, may comprise: a housing; a filter section which is provided at one end of the housing; a cavity section which is disposed upstream of the filter section within the housing and in which a functional material-holding member is accommodated; and a side air inlet which is formed at the outer side of the housing, corresponding to the cavity section, wherein a rotational airflow is supplied to the functional material-holding member through the side air inlet.

## Description

### TECHNICAL FIELD

The following embodiments relate to an inhaler.

### BACKGROUND ART

Research has been conducted on an inhaler that directly delivers a target material to the lungs of a user. For example, Korean Patent Application Publication No.10-2016-0065204 discloses a dry powder inhaler.

### DISCLOSURE OF THE INVENTION

### TECHNICAL GOALS

An inhaler according to an embodiment may inhale functional material powder over multiple times rather than consuming the functional material powder at once.

An inhaler according to an embodiment may uniformly transfer functional material powder.

An inhaler according to an embodiment may induce smooth discharge of functional material powder.

An inhaler according to an embodiment may be easily carried.

### TECHNICAL SOLUTIONS

An inhaler according to an embodiment may include a housing, a filter section provided at an end portion of the housing, a cavity section disposed in the housing on an upstream side of the filter section and accommodating a functional material accommodating member, and a side air inlet formed on an outer side of the housing corresponding to the cavity section, wherein rotating airflow may be provided to the functional material accommodating member via the side air inlet.

In an embodiment, the inhaler may further include a porous medium section disposed in the housing on an upstream side of the cavity section, wherein the porous medium section may communicate with the other end portion of the housing, and upstream airflow may be provided to the functional material accommodating member via the porous medium section.

Flavor powder may be impregnated into the porous medium section. Alternatively, a flavor capsule that is crushable may be accommodated in the porous medium section.

In an embodiment, a plurality of side air inlets may be provided.

A center of the side air inlet may be formed at a position 29% to 31% in a longitudinal direction from the end portion of the housing.

Three of the side air inlets are provided.

In an embodiment, the filter section may include a tube filter.

In an embodiment, the functional material accommodating member may include a capsule, and the capsule may include dry powder.

The dry powder may include one of nicotine, taurine, a pharmacological material, a flavor material, or a sweetener, or a combination thereof.

According to an embodiment, an inhalation device for inhaling a functional material may include an inhaler including a capsule filled with a functional material, a holder accommodating at least a portion of the inhaler and including a piercing member that forms a perforation in the capsule, wherein a plurality of side air inlets may be provided in the inhaler, and the side air inlet may be provided at a position 29% to 31% along a longitudinal direction from upstream of the inhaler.

In an embodiment, three of the side air inlets may be provided.

### EFFECTS OF THE INVENTION

According to an embodiment, functional material powder may not be consumed all at once and may be inhaled over multiple times.

According to an embodiment, functional material powder may be uniformly transferred.

According to an embodiment, smooth discharge of functional material powder may be induced.

According to an embodiment, an inhaler may be easily carried.

The effects of the inhaler according to embodiments are not limited to the above-mentioned effects, and other unmentioned effects can be clearly understood from the following description by one of ordinary skill in the art.

### BRIEF DESCRIPTION OF DRAWINGS

FIG. 1 is a front view of an inhaler according to an embodiment.
FIG. 2 is a perspective view illustrating an inside of an inhaler of an embodiment.
FIGS. 3A, 3B, and 3C are structural diagrams schematically illustrating an inhaler according to an embodiment.
FIG.4 shows a result of evaluating the amount of transfer of an inhaler of an embodiment.
FIG. 5 illustrates an inhalation device according to an embodiment.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, the expression "at least one of a, b, or c" should be construed as including a, b, c, a and b, a and c, b and c, or a, b, and c.

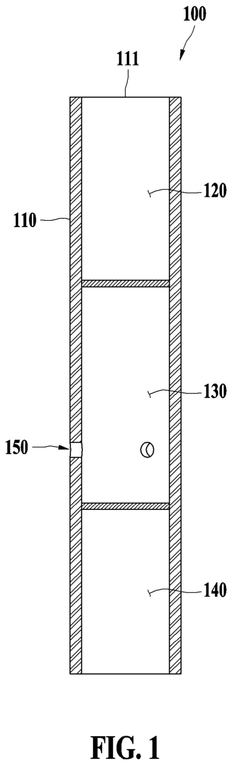

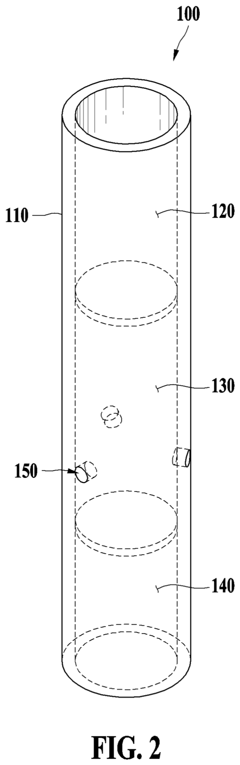

FIG. 1 is a front view of an inhaler 100 according to an embodiment, and FIG. 2 is a perspective view of an inside of the inhaler 100 according to an embodiment.

Referring to FIGS. 1 and 2, the inhaler 100 according to an embodiment may deliver a functional material to a user. For example, the inhaler 100 according to an embodiment may include an inhaler that delivers a dried pharmacological material or nicotine to a lung of a user in the form of powder or aerosol.

According to an embodiment, the inhaler 100 may include a housing 110, a filter section 120, a cavity section 130, a porous medium section 140, and a side air inlet 150.

For example, the housing 110, the filter section 120, the cavity section 130, the porous medium section 140 may be integrally formed as one, but the housing 110, the filter section 120, the cavity section 130, the porous medium section 140 may be separately formed and may be fixed to each other by bonding, such as hot melt or ethylene vinyl acetate (EVA) copolymer, or other eco-friendly adhesives.

The housing 110 may form an outer shell, may have a cylindrical shape, and may include a mouthpiece 111 that contacts a mouth part of a user at an end thereof. For example, a diameter of the housing 110 may be 7 mm, 8 mm, or 9 mm, and a length of the housing 110 may be 48 mm, 60 mm, or 84 mm.

The porous medium section 140, the cavity section 130, and the filter section 120 may be sequentially disposed in a direction from the front (e.g., an opposite side of the mouthpiece 111) of the housing 110 to the rear. For example, the porous medium section 140, the cavity section 130, the filter section 120 may be formed to communicate with each other.

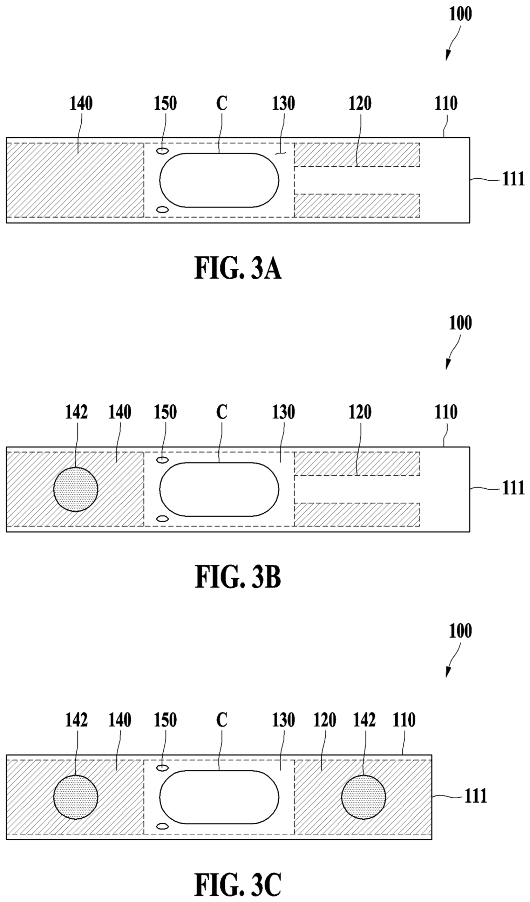

A functional material accommodating member (e.g., a capsule C of FIGS. 3A and 3B) may be accommodated in the cavity section 130, and the side air inlet 150 may be formed on an outer side of the housing 110 of the cavity section 130.

FIG. 3A is a schematic structural diagram of the inhaler 100 according to an embodiment, and FIG. 3B illustrates a structure in which a flavor capsule 142 is added to the inhaler 100 illustrated in FIG. 3A. FIG. 3C illustrates a structure in which a porous filter is applied to an open portion in the inhaler 100 illustrated in FIG. 3B.

Referring to FIG. 3A, the porous medium section 140 may include a cellulose acetate tow. Alternatively, the porous medium section 140 may include a biodegradable material.

The porous medium section 140 may be disposed to communicate with an open end portion of the housing 110, and upward airflow may be provided to the cavity section 130 via the porous medium section 140 from the open end portion of the housing 110. The functional material accommodating member (e.g., the capsule C of FIGS. 3A to 3C) may be elevated in the cavity section 130 by the upward airflow.

In an embodiment, flavor powder may be impregnated into the porous medium section 140, and as the upward airflow passes through the porous medium section 140, a flavor material in the flavor powder may be transferred to the mouthpiece 111 together with the upward airflow.

In an embodiment, the filter section 120 may include a tube filter.

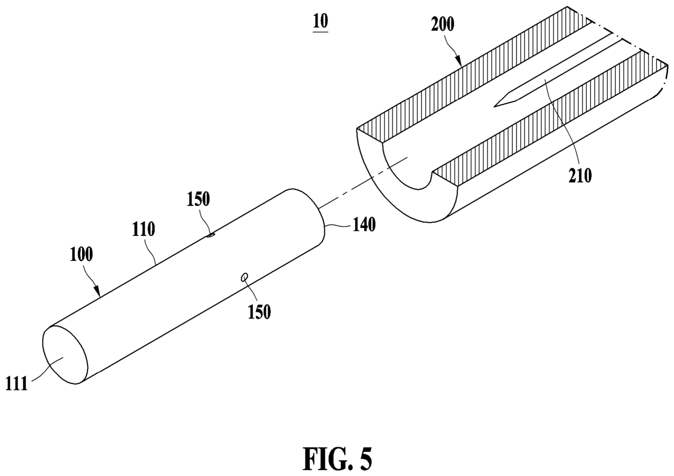

Referring to FIG. 3B, a flavor capsule 142 may be included in the porous medium section 140. For example, the flavor capsule 142 may be disposed in a center of a porous medium and may be crushed by a user. Alternatively, the flavor capsule 142 may be crushed by a capsule C by a piercing member (e.g., a piercing member 210 of FIG. 5).

The flavor capsule 142 may contain flavor powder or flavor liquid. For example, the flavor powder or flavor liquid may include an essential oil, menthol, and the like, and may include a natural or synthetic flavor material.

Referring to FIG. 3C, the filter section 120 may include a filter including a cellulose acetate tow and may have a porosity that is greater than a porosity of the porous medium section 140. In this case, the flavor capsule 142 may be disposed in the filter section 120, and the flavor capsule 142 may be crushed by the user. Although FIG. 3 illustrates that the flavor capsule 142 is included in both the porous medium section 140 and the filter section 120, the example is not limited thereto, and for example, the flavor capsule 142 may be included only in the porous medium section 140 or the flavor capsule 142 may be included only in the filter section 120.

For example, when the filter section 120 includes the filter including the cellulose acetate tow as shown in FIG. 3C, a recess structure may be further included in downstream of the filter section 120.

Referring to FIGS. 3A to 3C, the functional material accommodating member may include the flavor capsule C including dry powder. For example, the dry powder may be a pharmacological material, such as cannabidiol (CBD), a functional material, such as caffeine and taurine, or nicotine (free-base or nicotine salt). The dry powder may include a small amount of a flavor material, such as menthol or saccharin, or other sweeteners.

A size of the dry powder may be less than or equal to 10 µm in an aerodynamic diameter, and an average diameter thereof may be between 2.5 µm and 5 µm to deliver the dry powder to the bronchi and lungs. On the other hand, a size of the flavor material or other sweetener in the dry powder may be between 20 µm and 150 µm.

An amount of dry powder filling the capsule C may be between 10 mg and 100 mg, and preferably, about 30 mg.

An amount of dry powder transfer may be about 3 mg per breath, but since the amount depends on a respiratory flow of the user, the example is not limited thereto.

In an embodiment, the side air inlet 150 may be formed near an upstream side (e.g., an opposite side of the mouthpiece 111) of the cavity section 130 accommodating the capsule C. The side air inlet 150 may include a hole penetrating the inside of the housing 110 from the outside of the housing, and rotational airflow may be provided to the cavity section 130 from the outside of the housing 110 via the side air inlet 150. The number of side air inlets 150 may be two or more.

For example, the side air inlet 150 may be implemented by laser perforation, plasma perforation, mechanical perforation, or other physical or chemical perforation methods.

When the user bites the mouthpiece 111 and begins breathing, upward airflow may be provided to the cavity section 130 via the porous medium section 140, rotational airflow may be provided to the cavity section 130 via the side air inlet 150, and accordingly, elevation and rotation of the capsule C in the cavity section 130 may be induced.

In an embodiment, the side air inlet 150 may be formed at a position 29% to 31% in a longitudinal direction from an end portion (e.g., an end portion that is opposite to the mouthpiece 111) of the housing 110. Preferably, the number of side air inlets 150 may be three. A detailed description thereof is provided below.

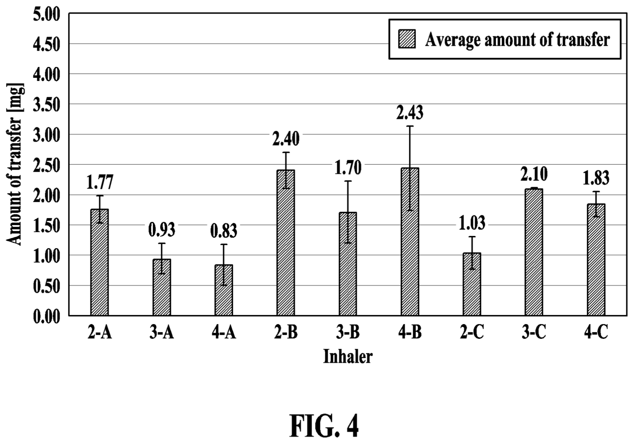

FIG. 4 is a graph showing an amount of dry powder transfer according to the number and positions of the side air inlets 150, Table 1 below shows setting conditions of experimental examples, and Table 2 shows an error value of the amount of dry powder transfer in each experimental example.

**[Table 1] Evaluation of Amount of Transfer**

| | Classification | | | | | | | | |
|---|---|---|---|---|---|---|---|---|---|
| Experimental example | 2-A | 3-A | 4-A | 2-B | 3-B | 4-B | 2-C | 3-C | 4-C |
| Number | 2 | 3 | 4 | 2 | 3 | 4 | 2 | 3 | 4 |
| Position (height) | 12.5 mm | | | 13.5 mm | | | 14.5 mm | | |

**[Table 2] Error Value**

| | |
|---|---|
| 2-A | 0.235702 |
| 3-A | 0.249444 |
| 4-A | 0.329983 |
| 2-B | 0.294392 |
| 3-B | 0.509902 |
| 4-B | 0.694422 |
| 2-C | 0.262467 |
| 3-C | 0 |
| 4-C | 0.20548 |

In each experimental example of Table 1, while a total length of the housing is set to 48 mm, the positions and number of side air inlets 150 may be differently set from an end portion in an upstream side (e.g., an opposite side of the mouthpiece) of the housing. In this case, a hole radius of the side air inlet 150 may be set to 0.5 mm, and the position in each experimental example may show a center position of the hole. The dry powder for inhalation is lactose monohydrate, and the capsule C is filled with 30 mg of the dry powder. A 0.9 mm perforation is formed in the capsule C, and a test is conducted based on 14 puffs of inhalation.

When one set includes 14 puffs, after conducting multiple sets of experiments, the error value of Table 2 shows a degree of an error among the sets. For example, each experimental example is repeated three times, and the error values of Table 2 are results obtained by repeating three times.

A bar graph extending along a vertical axis in FIG. 4 shows an average amount of transfer, and bars extending vertically from a peak of the bar graph show the error values of Table 2.

Referring to FIG. 4 and Tables 1 and 2, according to experimental examples 2-A, 3-A, and 4-A, the amount of dry powder transfer is not relatively large.

According to experimental examples 2-B, 3-B, and 4-B, the overall amount of transfer is highest, but the error values are large, and thus, it may be difficult to ensure uniform transfer.

According to experimental examples 2-C, 3-C, and 4-C, the amount of transfer is a satisfactory level, and the error values are also the smallest.

Accordingly, as the side air inlet 150 is positioned at a height of 14 mm to 15 mm from the end portion of the housing 110, in other words, at a height of 29% to 31%, a sufficient amount of dry powder transfer may be ensured while ensuring the uniformity of transferred dry powder.

Specifically, referring to Table 2, the error value of experimental example 3-C shows the smallest error value. Accordingly, it may be preferable that the side air inlet 150 is positioned at a height of 29% to 31% from the end portion of the housing 110 and the number thereof is three.

FIG. 5 illustrates an inhalation device 10 according to an embodiment.

Referring to FIG. 5, the inhalation device 10 according to an embodiment may include the inhaler 100 and a holder 200.

In an embodiment, at least a portion of the inhaler 100 according to an embodiment may be accommodated in the holder 200, and the holder 200 may include an elongated cavity having a shape corresponding to the housing 110 that forms an exterior of the inhaler 100. An end portion of the elongated cavity may be open, the other end portion may be closed, and a piercing member 210 may be positioned at the other end portion that is closed.

In an embodiment, the piercing member 210 may be disposed to protrude from the other end portion of the elongated cavity toward an opening and may have a length reaching the cavity section 130 of the inhaler 100.

For example, when the inhaler 100 is inserted into the holder 200, at least a portion of the capsule C in the inhaler 100 may be perforated by the piercing member 210. When the inhaler 100 is inserted into the holder 200, the porous medium section 140 of the inhaler 100 may be oriented to the elongated cavity. Alternatively, the mouthpiece 111 of the inhaler 100 may be oriented to the elongated cavity.

Next, while the inhaler 100 is completely or at least partially separated from the holder 200, the user may begin inhaling by biting the mouthpiece 111. Alternatively, the user may also inhale while the inhaler 100 is inserted into the holder 200, and in this case, a separate inlet through which air may be introduced may be formed in the holder 200.

The inhaler 100 according to an embodiment may enable inhalation of dry powder over multiple times by using inhalation of the user. This may lead to an advantage that the dry powder is not completely consumed at once.

Additionally, the inhaler 100 according to an embodiment may enable sufficient and uniform transfer of dry powder due to the optimal position and number of side air inlets 150.

The descriptions of the above-described embodiments are merely examples, and it will be understood by one of ordinary skill in the art that various changes and equivalents may be made thereto. Therefore, the scope of the disclosure should be defined by the appended claims, and all differences within the scope equivalent to those described in the claims will be construed as being included in the scope of protection defined by the claims.

## Claims

1. An inhaler for inhaling a functional material, the inhaler comprising:
a housing;
a filter section provided at an end portion of the housing;
a cavity section disposed in the housing on an upstream side of the filter section and accommodating a functional material accommodating member; and
a side air inlet formed on an outer side of the housing corresponding to the cavity section,
wherein rotating airflow is provided to the functional material accommodating member via the side air inlet.

2. The inhaler of claim 1, further comprising:
a porous medium section disposed in the housing on an upstream side of the cavity section,
wherein the porous medium section communicates with the other end portion of the housing, and upstream airflow is provided to the functional material accommodating member via the porous medium section.

3. The inhaler of claim 2, wherein flavor powder is impregnated into the porous medium section.

4. The inhaler of claim 2, wherein a flavor capsule that is crushable is accommodated in the porous medium section.

5. The inhaler of one of claims 1 to 4,
wherein a plurality of side air inlets is provided.

6. The inhaler of claim 5, wherein a center of the side air inlet is formed at a position 29% to 31% in a longitudinal direction from the end portion of the housing.

7. The inhaler of claim 6, wherein three of the side air inlets are provided.

8. The inhaler of one of claims 1 to 4, wherein the filter section comprises a tube filter.

9. The inhaler of one of claims 1 to 4, wherein the functional material accommodating member comprises a capsule, and the capsule comprises dry powder.

10. The inhaler of claim 9, wherein the dry powder comprises one of nicotine, taurine, a pharmacological material, a flavor material, or a sweetener, or a combination thereof.

11. An inhalation device for inhaling a functional material, the inhalation device comprising:
an inhaler comprising a capsule filled with a functional material;
a holder accommodating at least a portion of the inhaler and comprising a piercing member that forms a perforation in the capsule,
wherein a plurality of side air inlets is provided in the inhaler, and the side air inlets are provided at a position 29% to 31% along a longitudinal direction from upstream of the inhaler.

12. The inhaler of claim 11, wherein three of the side air inlets are provided.
